(19) Europäisches Patentamt
European Patent Office
Office européen des brevets

(11) Veröffentlichungsnummer: **0 110 869**
**B1**

(12) # EUROPÄISCHE PATENTSCHRIFT

(45) Veröffentlichungstag der Patentschrift: **18.03.87**

(51) Int. Cl.⁴: **C 07 D 333/28**, C 07 D 333/24 // A61K31/38

(21) Anmeldenummer: **83890206.2**

(22) Anmeldetag: **11.11.83**

(54) **Thienylessigsäureamidderivate und pharmazeutisch verträgliche Säureadditionssalze hiervon sowie Verfahren zu deren Herstellung.**

(30) Priorität: **26.11.82 AT 4308/82**

(43) Veröffentlichungstag der Anmeldung: **13.06.84 Patentblatt 84/24**

(45) Bekanntmachung des Hinweises auf die Patenterteilung: **18.03.87 Patentblatt 87/12**

(84) Benannte Vertragsstaaten: **BE CH DE FR GB IT LI LU NL SE**

(56) Entgegenhaltungen:
**DE-A-2 749 950**

**JOURNAL OF MEDICINAL CHEMISTRY, Band 25, Nr. 10, Oktober 1982, Seiten 1125-1126 J. SZMUSZKOVICZ et al.: "Benzeneacetamide amines: Structurally novel non-mmu opioids"**
**Medicinal Chemistry, 3 Auflage, Teil I, S.77**

**Die Akte enthält technische Angaben, die nach dem Eingang der Anmeldung eingereicht wurden und die nicht in dieser Patentschrift enthalten sind.**

(73) Patentinhaber: **Laevosan- Gesellschaft m.b.H., Estermannstrasse 17, A-4020 Linz (AT)**

(72) Erfinder: **Binder, Dieter, Univ. Prof., Dr., Sieveringerstrasse 207, A-1190 Wien (AT)**

(74) Vertreter: **Pawloy, Heinrich, Dr., Patentanwälte Dr. Heinrich Pawloy Dipl.- Ing. Helmut Sonn Dipl.- Ing. Arnulf Weinzinger Riemergasse 14, A-1010 Wien (AT)**

EP 0 110 869 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue therapeutisch wertvolle Derivate von Thienylessigsäureamiden der allgemeinen Formel

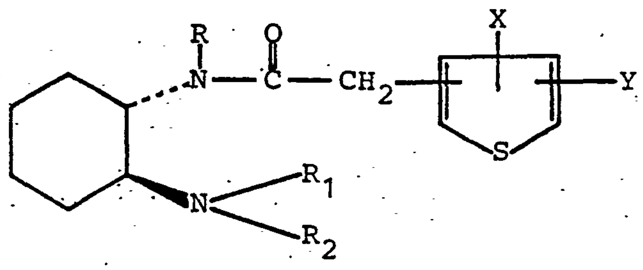

, (I)

worin die zwei Stickstoffe am Cyclohexanring transverknüpft sind, der basische Essigsäureamidrest in Stellung 2 oder 3 des Thiophenkerns steht, R $C_1$-$C_3$-Alkyl bedeutet, $R_1$ und $R_2$ unabhängig voneinander $C_1$-$C_3$-Alkyl oder zusammen mit dem Stickstoffatom, an das sie gebunden sind, einen Pyrrolidin- oder Piperidinring darstellen und X und Y je nach der Stellung der basischen Essigsäureamidgruppe in Stellung 2 bis 5 des Thiophenkerns unabhängig voneinander Wasserstoff, Chlor oder Brom bedeuten, und ihre pharmazeutisch verträglichen Säureadditionssalze sowie ein Verfahren zu deren Herstellung.

Die neuen Verbindungen der allgemeinen Formel (I) zeigen in verschiedenen Tiermodellen starke analgetische Eigenschaften, ohne physische Abhängigkeit (Sucht) zu verursachen.

Sie eignen sich daher hervorragend zur Langzeitbehandlung verschiedener Schmerzzustände.

In der DE-A- 2 749 950 und in J.Med.Chem. Bd. 25, Nr. 10, Seiten 1125-1126 sind bereits Verbindungen ähnlicher Struktur mit analgetischer Wirksamkeit beschrieben, die jedoch an Stelle des Thienylringes einen Phenylring aufweisen. Es hat sich nun überraschenderweise gezeigt, daß durch den Ersatz des Phenylringes durch einen Thienylring Verbindungen erhalten werden können, die eine stärkere analgetische Wirksamkeit aufweisen und trotzdem auch bei längerer Einnahme nicht zur Süchtigkeit führen.

Das erfindungsgemäße Verfahren ist dadurch gekennzeichnet, daß man eine Verbindung der allgemeinen Formel

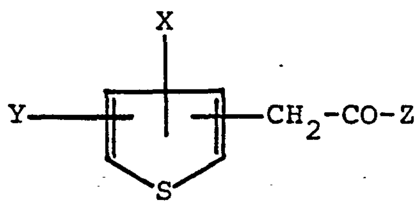

, (II)

worin der Essigsäurehalogenidrest in Stellung 2 oder 3 des Thiophenkerns steht, X und Y die obige Bedeutung haben und Z Chlor oder Brom bedeutet, mit einem trans-Cyclohexan-1,2-diamin der allgemeinen Formel

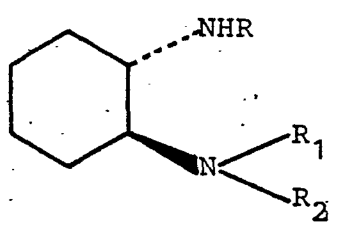

, (III)

worin R, $R_1$ und $R_2$ die obige Bedeutung haben, umsetzt.

Die Säureamidbildung wird üblicherweise in einem reaktionsinerten Lösungsmittel, wie z.B. Chloroform, Methylenchlorid, Äther oder Dioxan durchgeführt. Man geht am besten so vor, daß man die Amine der Formel (III), vorzugsweise gemeinsam mit mindestens äquimolaren Mengen eines leicht flüchtigen, stark basischen Säurefängers, wie z.B. Triäthylamin, vorlegt und die Säurechloride der Formel (II), verdünnt mit dem verwendeten Lösungsmittel, in äquivalenten Mengen, vorzugsweise geringfügig im Überschuß bei Temperaturen um 20°C zutropft. Die Reaktion ist bei dieser Temperatur längstens nach 18 h komplett.

Zur Aufarbeitung wird die Reaktionslösung mit gesättigter Natriumbicarbonatlösung geschüttelt, die organische Phase abgetrennt, getrocknet und eingedampft. Durch Extrahieren des Rückstandes mit kochendem Äther und Filtrieren über Aktivkohle erhält man die rohe, ölige Base der Formel (I), die man zur weiteren Reinigung säulenchromatographieren (z.B: Kieselgel 60 Merck, Artikel 7734; Eluens: Benzol/Triäthylamin = 9: 1) oder in kristalline, pharmazeutisch verträgliche Säureadditionssalze, z.B. Hydrochloride, überführen kann, die dann durch Umkristallisieren gereinigt werden können.

Dazu löst man die rohe Base der Formel (I) in einem geeigneten Lösungsmittel, z.B. einem niederen Alkohol

oder Wasser, gibt eine äquivalente Protonensäure zu, dampft im Vakuum das Lösungsmittel ab und kristallisiert den Rückstand aus Methanol, gegebenenfalls unter Zusatz von Äther um, oder man löst die rohe freie Base der Formel (I) in Äther oder Benzol, fällt die Säureadditionssalze durch Zugabe der entsprechenden Protonensäure aus und kristallisiert, wie oben angegeben, um. Geeignete Beispiele für derartige pharmazeutisch verträgliche Salze sind neben dem Salz der Salzsäure das Salz der Schwefelsäure, das Salz der Salpetersäure, das Salz der Phosphorsäure, Salze von Sulfonsäuren, das Salz der Benzoesäure, das Salz der Bernsteinsäure, das Salz der Maleinsäure, das Salz der Weinsäure und das Salz der Zitronensäure.

Die erfindungsgemäß erhältlichen Säureadditionssalze haben eine ebenso große analgetische Wirkung wie die entsprechenden freien Basen der Formel (I).

Die Verbindungen der Formel (III) sind literaturbekannt. Die Thienylessigsäurehalogenide der Formel (II) kann man, soweit sie nicht literaturbekannt sind, aus den entsprechenden Thienylessigsäuren (Formel (X)) auf eine dem Fachmann geläufige Weise, z.B. durch Erhitzen in überschüssigem Thionylchlorid oder -bromid erhalten.

Soweit die Thienylessigsäuren nicht literaturbekannt sind, kann man sie, ausgehend von literaturbekannten Thiophencarbonsäuren der Formel (IV), auf folgendem, dem Fachmann geläufigem Syntheseweg herstellen:

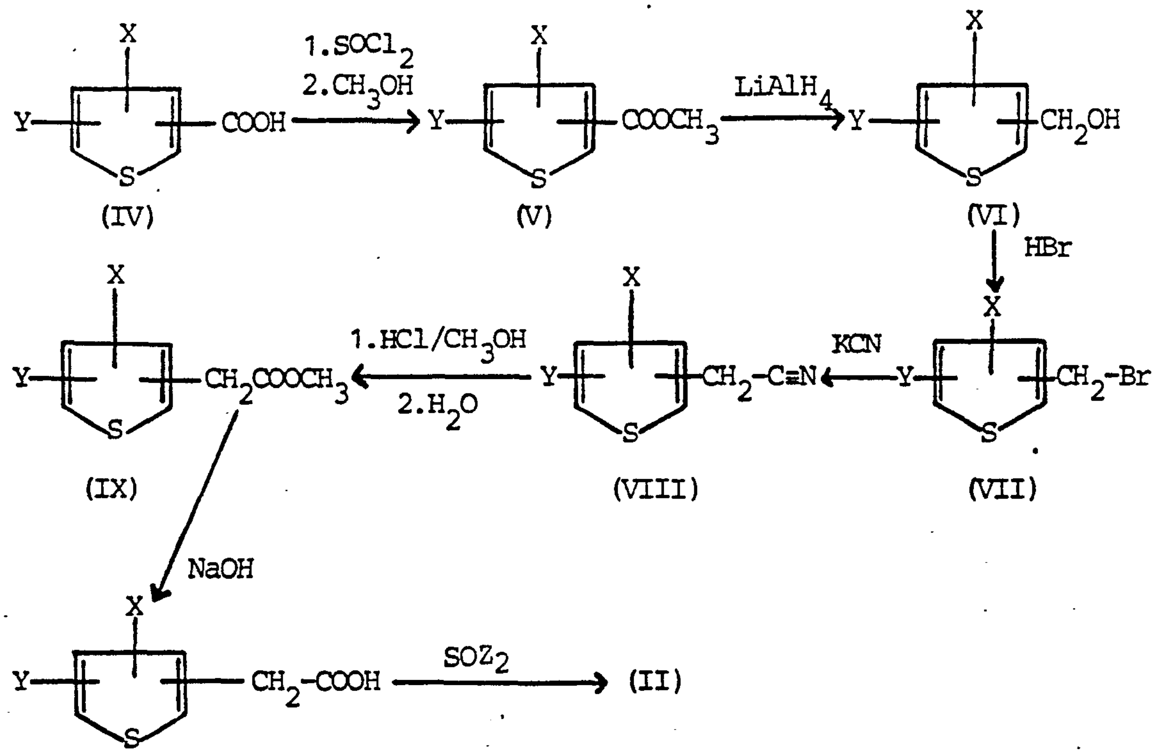

(IV) (V) (VI) (VII) (VIII) (IX) (X) (II)

Hierin haben X, Y und Z die obige Bedeutung.

Das folgende Beispiel soll die vorliegende Erfindung, ohne sie einzuschränken, erläutern:

**Beispiel:**

trans-N-Methyl-N-[2-(1-pyrrolidinyl)-cyclohexyl]-4,5-dichlor-2-thienylessigsäureamid-hydrochlorid (Formel I; R= $CH_3$, $-NR_1R_2$=

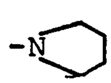,

X=4-Cl, Y=5-Cl)[LG-83-8-01]

Eine Lösung von 1,61 g (7,01 mMol) 4,5-Dichlor-2-thienyl-essigsäurechlorid (Formel II; X = 4-Cl, Y = 5-Cl, Z = Cl) in 15 ml abs. Chloroform wird während 15 min zu einer Lösung von 1,24 g (6,80 mMol) trans-N-Methyl-2-(1-pyrrolidinyl)-cyclohexylamin (Formel III; R=Methyl, $-NR_1R_2$=

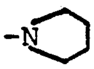 )

und 0,69 g (6,80 mMol) Triäthylamin in 60 ml abs. Chloroform bei Raumtemperatur zugetropft und noch 18 h bei Raumtemperatur nachgerührt. Dann wird die Reaktionslösung mit gesättigter Natriumhydrogencarbonatlösung ausgeschüttelt, die organische Phase mit Wasser gewaschen, über Natriumsulfat getrocknet und eingedampft. Der ölige Rückstand, bestehend aus der rohen Base der Formel (I) und etwas ätherunlöslichem Beiprodukt, wird mit 30 ml Äther aufgenommen, über Aktivkohle filtriert und in die Lösung HCl-Gas eingeleitet. Das kristallin ausfallende Hydrochlorid wird abgesaugt, in 4 ml Methanol gelöst, mit 50 ml feuchtem Äther ausgefällt, abgesaugt und der Vorgang nocheinmal wiederholt. Die farblosen Kristalle werden bei 13,3 Pa und Raumtemperatur 5 h getrocknet. Auf diese Weise erhält man das nichthygroskopische Hemihydrat des Hydrochlorids. Ausbeute: 57 %, Fp. 216-218°C. Die kristallwasserfreie Verbindung, die man beim Trocknen im Vakuum bei 13,3 Pa und 140°C erhält, ist hygroskopisch.

Analog kann man erhalten: trans-N-Methyl-N-[2-(1-pyrrolidinyl)-cyclohexyl]-5-chlor-2-thienylessigsäureamid-hydrochlorid (Formel (I); $R = CH_3$, $-NR_1R_2 =$

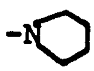,

X=H, Y=5-Cl), Fp. (Methanol/Äther): 175-178°C (60%), kristallwasserfreie Verbindung, farblose Kristalle, nicht hygroskopisch.

trans-N-Methyl-N-[2-(1-pyrrolidinyl)-cyclohexyl]-2,5-di-chlor-3-thienylessigsäureamid-hydrochlorid (Formel (I); $R = CH_3$, $-NR_1R_2 =$

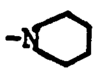,

X=2-Cl, Y=5-Cl), [LG-83-8-02], Fp. (Methanol/Äther): 252-254°C (55 %), kristallwasserfreie Verbindung, farblose Kristalle, nicht hygroskopisch.

Das Ausgangsmaterial kann wie folgt hergestellt werden: 4,5-Dichlor-2-thienylessigsäurechlorid (Formel (II);

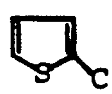,

X = Cl, Y = 5-Cl, Z = Cl)

1,48 g (7,01 mMol) 4,5-Dichlor-2-thienylessigsäure werden in 15 ml Thionylchlorid gelöst und 2 h rückflußerhitzt. Dann wird das überschüssige Thionylchlorid im Vakuum abdestilliert und das zurückbleibende Säurechlorid ohne weitere Reinigung in die nächste Stufe eingesetzt. Rohausbeute: fast quantitativ.

Analog werden hergestellt: 5-Chlor-2-thienylessigsäurechlorid, 2,5-Dichlor-3-thienylessigsäurechlorid.

Daß es sich bei den erfindungsgemäßen Verbindungen um K-spezifische Analgetika handelt, geht aus der pharmakologischen Untersuchung von zwei erfindungsgemäßen Verbindungen, nämlich LG 83-8-01 und LG 83-8-02, hervor:

Beide Verbindungen zeigten im Brennstrahl-Analgesietest an der Maus eine $ED_{50}$ von 0,48 mg/kg Körpergewicht bei einer $LD_{50}$ von etwa 50 mg/kg, wobei die typischen V-rezeptoragonistischen Eigenschaften, wie sie bei den Opiaten auftreten, nämlich das Straub'sche Schwanzphänomen, der gebogene Rücken und eine gesteigerte lokomotorische Aktivität nicht zu beobachten waren.

Die erfindungsgemäßen Verbindungen können in Form von Tabletten oder Kapseln, welche eine Einheitsdosis der Verbindungen zusammen mit Verdünnungsmitteln, wie Maisstärke, Kalziumcarbonat, Dikalziumphosphat, Alginsäure, Lactose, Magnesiumstearat, Primogel oder Talkum enthalten, oral appliziert werden. Die Tabletten werden in herkömmlicher Weise durch Granulieren der Inhaltsstoffe und Pressen, die Kapseln durch Einfüllen in Hartgelatinekapseln geeigneter Größe hergestellt.

Die erfindungsgemäßen Verbindungen können auch parenteral, beispielsweise durch intramuskuläre, intravenöse oder subkutane Injektion, appliziert werden. Für die parenterale Applikation werden sie am besten in Form einer sterilen wässerigen Lösung verwendet, welche andere gelöste Stoffe, wie tonische Mittel und Mittel zur Einstellung des pH-Wertes enthalten kann. Die Verbindungen können zu destilliertem Wasser zugesetzt werden und der pH-Wert kann unter Verwendung einer Säure, wie Citronensäure, Milchsäure oder

Salzsäure, auf 3 bis 6 einstellt werden. Ausreichend gelöste Stoffe, wie Dextrose oder Salzlösung, können zugesetzt werden, um die Lösung isotonisch einzustellen. Die erhaltene Lösung kann dann sterilisiert und in sterile Glasampullen geeigneter Größe, so daß sie das gewünschte Lösungsvolumen enthalten, eingefüllt werden. Die erfindungsgemäßen Verbindungen können auch durch Infusion einer parenteralen Formulierung, wie oben beschrieben, in eine Vene appliziert werden.

Für die orale Applikation beim Menschen wird angenommen, daß der tägliche Dosierungswert einer erfindungsgemäßen Verbindung im Bereich von 0,1 bis 10 mg/kg pro Tag für einen typischen erwachsenen Patienten von 70 kg liegt.

Selbstverständlich wird aber der Arzt in jedem Falls die tatsächliche Dosierung bestimmen, welche für den individuellen Patienten am geeignetsten ist, wobei diese mit dem Alter, der Masse und dem Ansprechen des Patienten variieren kann.

**Patentansprüche**

1. Thienylessigsäureamide der allgemeinen Formel

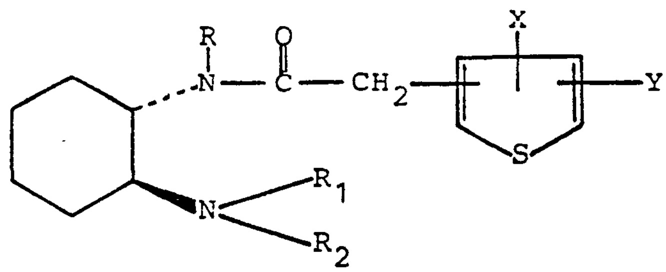

, (I)

worin die zwei Stickstoffe am Cyclohexanring transverknüpft sind, der basische Essigsäureamidrest in Stellung 2 oder 3 des Thiophenkerns steht, R $C_1$-$C_3$-Alkyl bedeutet, $R_1$ und $R_2$ unabhängig voneinander $C_1$-$C_3$-Alkyl oder zusammen mit dem Stickstoffatom, an das sie gebunden sind, einen Pyrrolidin- oder Piperidinring darstellen und X und Y je nach der Stellung der basischen Essigsäureamidgruppe in Stellung 2 bis 5 des Thiophenkerns unabhängig voneinander Wasserstoff, Chlor oder Brom bedeuten, und ihre pharmazeutisch verträglichen Säureadditionssalze.

2. trans-N-Methyl-N-[2-(1-pyrrolidinyl)-cyclohexyl]-4,5-dichlor-2-thienylessigsäureamid.

3. trans-N-Methyl-N-[2-(1-pyrrolidinyl)-cyclohexyl]-5-chlor-2-thienylessigsäureamid.

4. trans-N-Methyl-N-[2-(1-pyrrolidinyl)-cyclohexyl]-2,5-dichlor-3-thienylessigsäureamid.

5. Verfahren zur Herstellung der Verbindungen der Formel (I) und ihrer Säureadditionssalze, dadurch gekennzeichnet, daß man eine Verbindung der allgemeinen Formel

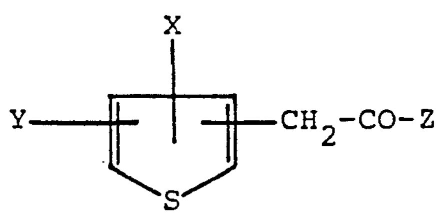

, (II)

worin der Essigsäurehalogenidrest in Stellung 2 oder 3 des Thiophenkerns steht, X und Y die obige Bedeutung haben und Z Chlor oder Brom bedeutet, mit einem trans-Cyclohexan-1,2-diamin der allgemeinen Formel

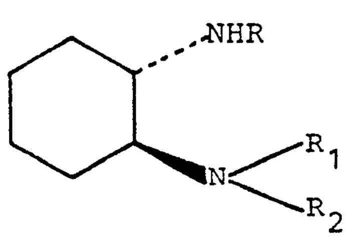

, (III)

worin R, $R_1$ und $R_2$ die obige Bedeutung haben, umsetzt und gegebenenfalls die erhaltenen Verbindungen der Formel (I) in ihre pharmazeutisch verträglichen Säureadditionssalze überführt.

6. Verfahren nach Anspruch 5, dadurch gekennzeichnet, daß man eine erhaltene Base der Formel (I) in ihr Hydrochlorid überführt.

**Revendications**

1 - Thiénylacétamides de formule générale:

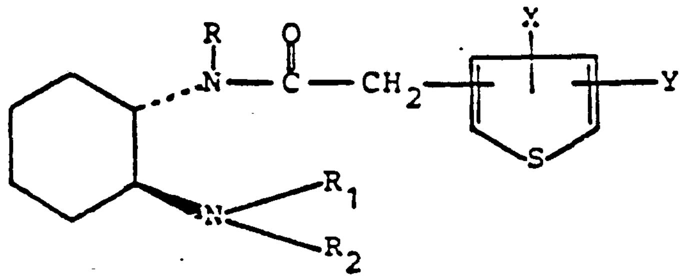

(I)

dans laquelle:

- les deux azotes sont liés suivant une configuration trans au noyau cyclohexane;
- le reste basique de l'acétamide se trouve en position 2 eu 3 du noyau thiophène;
- R signifie un reste alkyle en $C_1$-$C_3$;
- $R_1$ et $R_2$ représentent, indépendamment l'un de l'autre, un reste alkyle en $C_1$-$C_3$, ou, conjointement avec l'atome d'azote auquel ils sont liés, un noyau pyrrolidino ou pipéridino; et
- X et Y, se trouvant chacun dans l'une des positions 2 à 5 selon la position du reste basique de l'acétamide, signifient, indépendamment l'un de l'autre, hydrogène, chlore ou brome,

et leurs sels d'addition acide pharmaceutiquement acceptables.

2 - Trans-N-méthyl-N-[(pyrrolidinyl-1)-2 cyclohexyl] dichloro-4,5 thiénylacétamide-2.

3 - Trans-N-méthyl-N-[(pyrrolidinyl-1)-2 cyclohexyl] chloro-5 thiénylacétamide-2.

4 - Trans-N-méthyl-N-[(pyrrolidinyl-1)-2 cyclohexyl] dichloro-2,5 thiénylacétamide-3.

5 - Procédé de fabrication des composés de formule (I) et de leurs sels d'addition acide, caractérisé par le fait que l'on fait réagir un composé de formule générale:

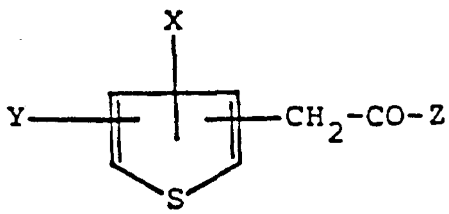

(II)

dans laquelle le reste de l'halogénure de l'acide acétique se trouve en position 2 ou 3 du noyau thiophéne,

- X et Y ont les significations indiquées ci-dessus; et
- Z représente chloro ou bromo,

avec une trans-cyclohexanediamine-1,2 de formule générale:

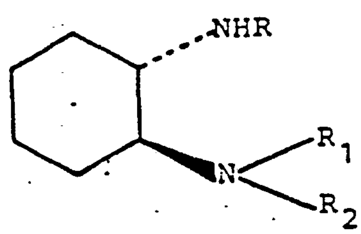

(III)

dans laquelle R, $R_1$ et $R_2$ ont les significations indiquées ci-dessus,

et, le cas échéant, on transforme les composés de formule (I) obtenus en leurs sels d'addition acide pharmaceutiquement acceptables.

6 - procédé selon la revendication 5, caractérisé par le fait que l'on transforme une base de formule (I) obtenue en son chlorhydrate.

**Claims**

1. Thienyl acetamides of the general formula

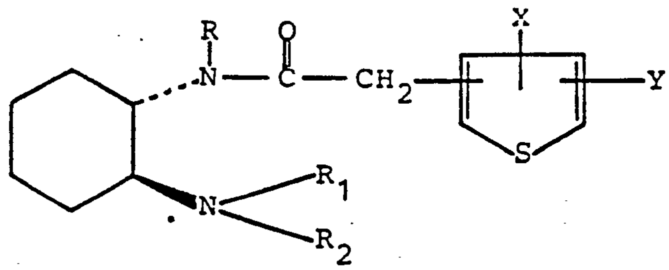

(I)

in which the two nitrogens are linked to the cyclohexane ring in trans configuration, the basic acetamide radical is in position 2 or 3 of the thiophene ring, R is $C_1$-$C_3$ alkyl, $R_1$ and $R_2$ are independently $C_1$-$C_3$ alkyl or together with the nitrogen atom to which they are bond form a pyrrolidine or piperidine ring and X and Y depending on the position of the basic acetamide group in position 2 to 5 of the thiophene ring are independently hydrogen, chlorine or bromine, and the pharmaceutically acceptable acid addition salts thereof.

2. Trans-N-methyl-N-[2- (1-pyrrolidinyl) -cyclohexyl]-4, 5-dichloro-2-thienyl acétamide.

3. Trans-N-methyl-N-[2- (1-pyrrolidinyl) -cyclohexyl]-5-chloro-2-thienyl acetamide.

4. Trans-N-methyl-N-[2- (1-pyrrolidinyl) -cyclohexyl] -2, 5-dichloro-3-thienyl acetamide.

5. A process for preparing the compounds of formula (I) and the acid addition salts thereof, which comprises reacting a compound of general formula

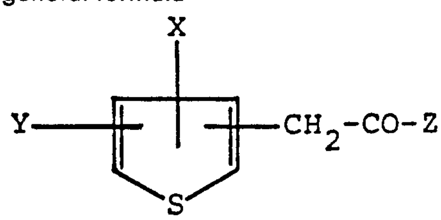

(II)

in which the acetic halide radical is in position 2 or 3 of the thiophene ring, X and Y are as defined above and Z is chlorine or bromine, with a trans-cyclohexane-1, 2-diamine of the general formula

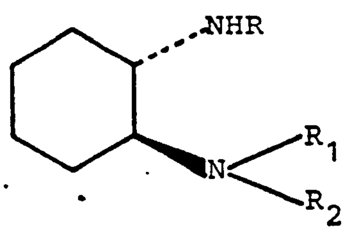

(III)

in which R, $R_1$ and $R_2$ are as defined above, and optionally converting the obtained compounds of formula (I) into their pharmaceutically acceptable acid addition salts.

6. The process of claim 5 in which an obtained base of formula (I) is converted into its hydrochloride.